Europäisches Patentamt

European Patent Office

Office européen des brevets

(11) Publication number: **0 266 472**
**A1**

## (12) EUROPEAN PATENT APPLICATION

(21) Application number: **86830318.1**

(22) Date of filing: **03.11.86**

(51) Int. Cl.⁴ **A61F 13/12**

(43) Date of publication of application:
**11.05.88 Bulletin 88/19**

(84) Designated Contracting States:
**DE FR GB IT**

(71) Applicant: **Ritorto, Salvatore**
**Via Gradoli, 65**
**I-00189 Roma(IT)**

(72) Inventor: **Ritorto, Salvatore**
**Via Gradoli, 65**
**I-00189 Roma(IT)**

(74) Representative: **Mascioli, Alessandro, Prof.Dr.**
**c/o A.N.D.I. Associazione Nazionale degli**
**Inventori Via Lima, 35**
**I-00198 Roma(IT)**

(54) **Scalp-raising device for the prevention of baldness.**

(57) Scalp-raising device for the prevention of bald-ness composed of a band of elastic material (2), encircling the base of the skull, and at least two elastic cross-bands (3), attached to the said band (2), and running above the patient's head.

*Fig. 1*

EP 0 266 472 A1

## SCALP-RAISING DEVICE FOR THE PREVENTION OF BALDNESS

This innovation concerns a device to prevent definitive hair loss, eliminate itching and reduce dandruff formation. The use of the device prevents the onset of the events underlying baldness.

Hair loss is a growing concern, and creates considerable problems, both psychological and aesthetic, for a great number of people.

A number of methods have been suggested to prevent hair loss or to re-implant hair subsequent to hair loss, involving the use of special lotions, particular types of treatment, or transplants, and these have turned out to be far from effective and in some case painful and expensive.

The basic reason for hair loss is the inadequate flow of blood and lymph, which supply hair with its basic nutrition. Two very important factors in maintaining good hair health are skin elasticity and skin mobility. Healthy skin can move freely on subcutaneous tissues, whereas the skin covering the skull, particularly in male individuals, tends to flatten and adhere increasingly to subcutaneous tissues. This is one of the main causes behind a reduction in blood flow which, depending on the individual concerned, leads to the skin becoming either dry or damp, and the formation of dandruff or hair loss.

In light of the above, there are clear advantage in a device of the kind covered by the present innovation, which confers uniform elasticity and thickness on the scalp and prevents the stoppage of the intercutaneous circulation on the upper areas of the skull.

One further aim of this innovation is to provide a device of this kind which is also capable of re-establishing the natural physiological equilibrium of the scalp so as to prevent hair loss and stop dandruff from occurring. This results are obtained, according to the applicant's proposals by using a device which is basically composed of a main band covering the base of the scalp, the forehead, the nape, and the ears, with cross-bands running above the patient's head.

The specific object of this invention is therefore a device for the prevention of hair loss and reduction of dandruff and itching made up off a band, made of elastic material, covering the base of the skull, and at least two elastic crossbands running above the patient's head.

Preferentially, according to the innovation, there should be two of these bands crossing centrally at a point corresponding to the top of the head.

Under the innovation, the main band and the cross-bands are to be in rubber latex.

This innovation will now be described indicatively but not exhaustively in the way represented in the annexed drawing which shows a perspective view of the divice according to the innovation.

The cross-bands (3) are attached at points (4) to the band (2) and cross at the point (5), which corresponds to the top of the skull, passing over the forehead, behind the nape and behind the ears.

When device (1) is worn, cross-bands (3) tend to raise the scalp, and restore a natural position so that the skin on the top of the head regains the same thinkness and elasticity existing at the base of the skull.

This skin is stretched when combing, because of the force of gravity and because of hormonal causes which modify skin elasticity - the final result, after a number of years, being the loss of few tenths of a millimetre of thickness, which are enough to generate hair loss.

The application of the device for just a few minutes a day for a month makes it possible to restore the original condition of the skin and to avoid hair loss.

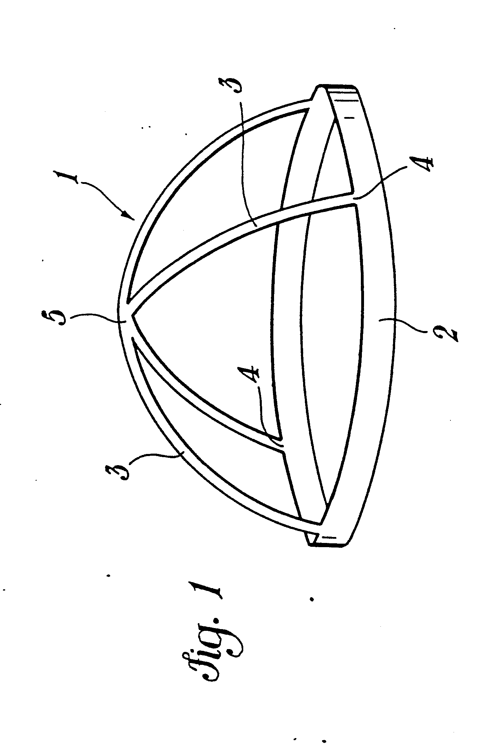

## Claims

1. Scalp raising device for the prevention of baldness characterized by the fact of being composed of a band of elastic material (2), encircling the base of the skull, and at least two elastic cross-bands (3), attached to the said band (2), and running above the patient's head.

2. Scalp raising device for the prevention of baldness according to claim 1, characterized by the fact that these cross-bands are in the number of two and cross at a point corresponding to the apex of the head.

3. Scalp raising device for the prevention of baldness according to claim 1 characterized by the fact that the above band and the above cross-bands are made of rubber latex.

4. Scalp raising device for the prevention of baldness according to each of the preceding claims, substantially as illustrated and described.

*Fig. 1*

0 266 472

European Patent Office

**EUROPEAN SEARCH REPORT**

Application number

EP 86 83 0318

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A- 906 781 (PERREAU) <br> * The whole document * | 1-3 | A 61 F 13/12 |
| | --- | | |
| X | US-A-1 908 669 (N.H.HORNE) <br> * Figure 1; page 2, lines 38-49 * | 1,3 | |
| | --- | | |
| A | US-A-2 825 328 (M.H.OLSEN) <br> * Figure 3 * | 1 | |
| | ----- | | |

TECHNICAL FIELDS SEARCHED (Int. Cl.4)

A 61 F

The present search report has been drawn up for all claims

| Place of search | Date of completion of the search | Examiner |
|---|---|---|
| THE HAGUE | 10-07-1987 | ARGENTINI A. |